(19) Europäisches Patentamt
European Patent Office
Office européen des brevets

(11) Publication number: 0 021 235
A1

# (12) EUROPEAN PATENT APPLICATION

(21) Application number: **80103223.6**

(22) Date of filing: **10.06.80**

(51) Int. Cl.³: **C 07 J 9/00**

(30) Priority: **14.06.79 US 48629**

(43) Date of publication of application: **07.01.81 Bulletin 81/1**

(84) Designated Contracting States: **CH DE FR GB LI NL**

(71) Applicant: **DIAMOND SHAMROCK CORPORATION, 1100 Superior Avenue, Cleveland Ohio 44114 (US)**

(72) Inventor: **Johnson, Richard L., 309 East Armitage, Northlake, Illinois (US)**
Inventor: **Hirsch, Arnold L., 609 N.Marion Street, Oak Park, Illinois (US)**

(74) Representative: **Weickmann, Heinrich, Dipl.-Ing Patentanwälte Dipl.Ing.H.Weickmann et al, Dipl.Phys.Dr.K.Fincke Dipl.Ing.F.A.Weickmann Dipl.Chem.B.Huber, Dr.-Ing.H.Liska Möhlstrasse 22, D-8000 München 86 (DE)**

(54) **Preparation of 25-hydroxycholesterol.**

(57) Improved yields of 25-hydroxycholesterol are obtained by employing 3 β-hydroxy-5-cholenic acid as the starting material. This compound is an intermediate for 25-hydroxycholecalciferol.

EP 0 021 235 A1

# PREPARATION OF 25-HYDROXYCHOLESTEROL

## BACKGROUND OF THE INVENTION

### 1. Field of the Invention

This invention relates to the preparation of 25-hydroxycholesterol from 3β-hydroxy-5-cholenic acid, hereinafter referred to as cholenic acid. The compound 25-hydroxycholesterol is subsequently converted to 25-hydroxycholecalciferol (25-OH-$D_3$) by conventional means.

### 2. Description of the Prior Art

The compound 25-hydroxycholecalciferol is characterized by vitamin D activity. It has been isolated from plasma of hogs which have been maintained on high daily levels of vitamin $D_3$ for extended periods as described in U.S. Patent No. 3,565,924 - DeLuca et al - February 23, 1971. Synthetic routes have been developed as well. See, e.g., U.S. Patent No. 3,772,361 - DeLuca et al - November 13, 1973 where 25-OH-$D_3$ is prepared from cholest-5,7-diene-3β, 25-diol, in turn prepared from 3,25-cholesteryl diacetate; U.S. Patent No. 3,833,622 - Babcock et al - September 3, 1974 where 3β-hydroxychol-5-en-24-oic acid is converted after protection of the 3-hydroxy group to its acid chloride in the initial steps of preparing 25-OH-$D_3$ and U.S. Patent No. 4,001,096 - Salmond - January 4, 1977 where 25-hydroxy previtamin $D_3$ is prepared via irradiation of cholesta-5,7-diene-3β, 25-diol. In U.S. Application Serial No. 877,569 - Hirsch et al, filed February 13, 1978 there is disclosed a process for converting cholenic acid to 25-hydroxycholesterol by application of the Willgerodt reaction.

Further, in a series of studies by Johnson et al, J. Med. Chem., 20,5 (1977) side chain analogs of 25-OH-$D_3$ have been prepared. The dinor analog and the homovitamin were prepared from 23,24-dinorcholenic acid and from 25-homo-3β-hydroxychol-5-en-25-oic acid respectively. In each instance, the starting material was converted to the methyl ester and the 3β-hydroxyl protected as the tetra hydropyranyloxy ether. Reduction with lithium aluminum hydride yielded the alcohol which was converted to the tosylate. Displacement of the tosylate with sodium cyanide gave the corresponding nitrile. Alkylation with methyl lithium followed by acid hydrolysis yielded the hydroxy ketone which was subsequently converted to the previtamin and then irradiated.

## SUMMARY OF THE INVENTION

25-Hydroxycholesterol is prepared from cholenic acid according to the following sequence of steps.

A. Cholenic acid is esterified.

B. The 3β-hydroxyl moiety is protected as an acid labile ether.

C. Reduction with a hydride reducing agent yields the primary alcohol.

D. The alcohol from C is converted into a leaving group.

E. Displacement of the leaving group with cyanide yields the nitril.

F. The nitrile is converted to the methyl ketone by C-alkylation with a methyl organometallic reagent followed by hydrolysis.

G. 25-Hydroxycholesterol is obtained by reaction of the methyl ketone with a methyl organometallic reagent.

To obtain 25-hydroxycholecalciferol, 25-hydroxycholesterol is brominated to its 7-bromo derivative followed by dehydrobromination to the corresponding 7-dehydro compound which is subject to ultraviolet irradiation. See. e.g., U.S. Patent No. 3,846,455 - Ikekawa et al - November 5, 1974.

Thus, it is the object of this invention to prepare 25-hydroxycholesterol more economically and in greater yields than heretofore obtained. The 25-hydroxycholesterol obtained can be converted by conventional means to 25-hydroxycholecalciferol thereby yielding 25-hydroxycholecalciferol in greater yields and at reduced cost.

The following table based upon the example illustrates schematically the process for preparing 25-hydroxycholesterol from cholenic acid. In both the table and example the numbers identify the starting material, intermediates and end product while the letters identify the reactions.

TABLE

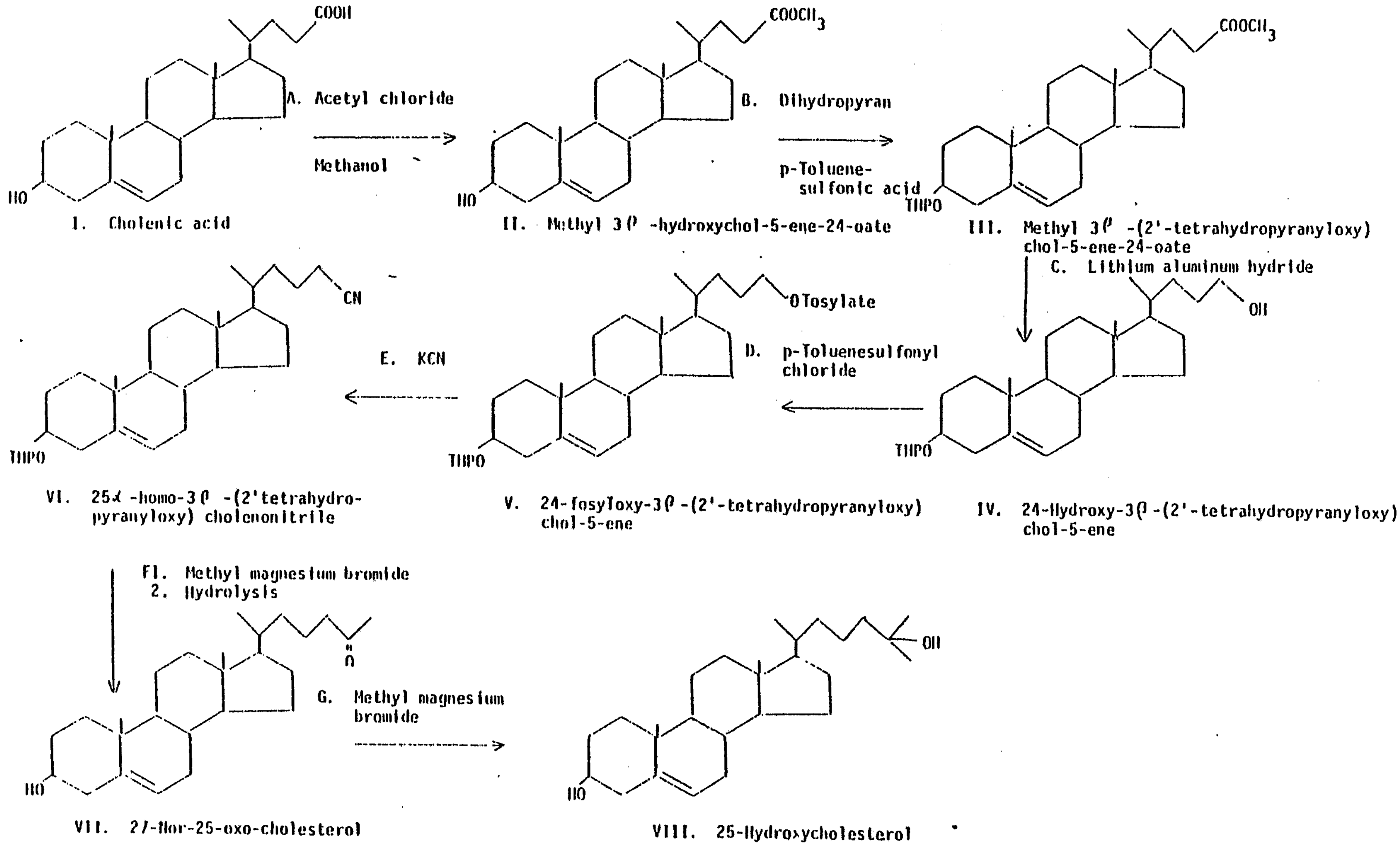


I. Cholenic acid

II. Methyl 3β -hydroxychol-5-ene-24-oate

III. Methyl 3β -(2'-tetrahydropyranyloxy) chol-5-ene-24-oate

IV. 24-Hydroxy-3β -(2'-tetrahydropyranyloxy) chol-5-ene

V. 24-Tosyloxy-3β -(2'-tetrahydropyranyloxy) chol-5-ene

VI. 25α -homo-3β -(2'tetrahydro-pyranyloxy) cholenonitrile

VII. 27-Nor-25-oxo-cholesterol

VIII. 25-Hydroxycholesterol

## DESCRIPTION OF THE PREFERRED EMBODIMENTS

With respect to Step A, esterification of cholenic acid can be achieved by reacting cholenic acid with any aliphatic short chain alcohol such as methanol, ethanol, propanol, etc. in great excess. The excess methanol over stoichiometric amounts serves as the solvent. As catalyst, any acid source is effective, e.g., acetyl chloride, phosphorous oxychloride, paratoluenesulfonic acid, hydrogen chloride (gas) and the like. Useful inert solvents for the reaction are aromatic hydrocarbons such as benzene and toluene, halogenated hydrocarbons such as methylene chloride, ethylene dichloride, etc., and ethers such as dioxane and tetrahydrofuran. Reaction temperature can vary from ambient to reflux temperature.

With respect to Step B, protection of the 3β-hydroxy substituent can be achieved by protection as an acid labile ether using e.g., cycloalkenyl ethers such as dihydropyran, mixed acetals or other base stable protecting compound. Generally the ether, acetal, etc., is present in from about 1.5 to about 4 moles per mole of starting ester. Useful inert solvents for the reaction are aromatic hydrocarbons such as benzene and toluene, aliphatic hydrocarbons such as hexane and pentane; halogenated hydrocarbons such as methylene chloride, ethylene dichloride, etc.; ethers such as diethylether, dioxane and tetrahydrofuran. As catalyst, any acid source is effective such as hydrochloric acid, paratoluenesulfonic acid, phosphorous oxychloride, etc. Reaction is carried out at temperatures between room temperature and reflux.

With respect to Step C, reduction to alcohol can be achieved by reaction with any convenient hydride reducing agent such as lithium aluminum hydride, lithium borohydride, lithium tritertiarybutoxy aluminum hydride, etc., in an appropriate aprotic solvent such as ethers (tetrahydrofuran) and aromotic hydrocarbons (benzene, toluene) at low temperature to reflux using from about 0.5 to about 2.5 moles of hydride reducing agent per mole of ester.

With respect to Step D, the alcohol can be converted to any good leaving group such as mesylate, tosylate, etc., at room temperature or below using from about 1.5 to about 2 molar excess of leaving group with a small amount of pyridine as solvent.

With respect to Step E, formation of the nitrile can be achieved by reacting with inorganic cyanides such as sodium cyanide, potassium cyanide, etc., in aprotic solvents such as dimethylformamide, dimethylactetamide, dimethylsulfoxide, etc., at room temperature to reflux. A large excess of cyanide, e.g., from about a 5 to about a 10 fold molar excess is used. It is, of course, essential that the cyanide be at least slightly soluble in the solvent.

With respect to Step F, methyl ketone formation can be achieved by reaction with from about a 5% to about a 10% molar excess of methyl organometallic reagent, e.g., methyl magnesium bromide, methyl magnesium iodide, methyl magnesium chloride and methyl lithium. Methyl lithium is the preferred reagent. Reaction is carried out at temperatures of -20°C to reflux. Useful solvents for the reaction are aromatic hydrocarbons such as benzene and toluene, aliphatic hydrocarbons such as hexane and heptane and ethers such as dioxane and tetrahydrofuran. Thereafter, acidic hydrolysis is carried out. Simultaneously, the acid labile ether is hydrolyzed thereby regenerating the 3 β -hydroxy group.

With respect to Step G, formation of 25-hydroxycholesterol can be achieved by reaction of the methyl ketone with from about a 5% to about a 10% molar excess of methyl organometallic reagent, e.g., methyl magnesium bromide, methyl magnesium iodide, methyl magnesium chloride and methyl lithium. Methyl lithium is the preferred reagent. Reaction is carried out at temperatures of -20°C to reflux. Useful solvents are aromatic hydrocarbons such as benzene and toluene, aliphatic hydrocarbons such as hexane and heptane and ethers such as dioxane and tetrahydrofuran.

For a fuller understanding of this invention, reference may be made to the following examples. These examples are given merely to illustrate the invention and are not to be construed in a limiting sense.

EXAMPLE

(A) Methyl 3 β -hydroxychol-5-ene-24-oate (II)

Cholenic acid, 100 g (0.267 moles), was slurried in 1200 ml of a methanol:toluene mixture (5:1). Acetyl chloride, 39 g (0.50 mole), was added dropwise to the stirred mixture during 30 minutes.

The slurry cleared to a murky, pale copper colored solution within 15 minutes. After stirring 1-1.5 hours the solution was filtered and concentrated in vacuo to 300 ml. Methanol (300 ml) was added and the resulting ester was recrystallized from hot solution giving 100 g of the title compound (96.4%):mp 146-147.5°C; nmr $\tau$ 4.54-4.73 ($H_6$,m), 6.00-6.95 and 6.32 ($H_{3\alpha}$ ,br m and $CO_2CH_3$ superimposed s), 8.97 ($C_{19}CH_3$,s), 9.10 ($C_{21}CH_3$,d,J $\simeq$ 5Hz), 9.30 ($C_{18}CH_3$,s).

(B) Methyl 3$\beta$-(2'tetrahydropyranyloxy)-chol-5-ene-24-oate (III)

The following was carried out to protect the 3$\beta$-hydroxy moiety of II as the tetrahydropyranyloxy ether. The methyl cholenate (II), 20g (0.052 moles), was slurried in 180 ml dioxane containing 300 mg of para toluene sulfonic acid monohydrate. Dihydropyran, 20 ml (0.22 moles), was added to the stirred mixture in 5 minutes. After 30 minutes the entire reaction mixture was poured into aqueous saturated sodium bicarbonate solution and extracted with ether. The ether layer was dried and concentrated to dryness in vacuo. The fluffy white solid was recrystallized from methanol containing 1% pyridine affording 22.6 g (88%) of the desired tetrahydropyranyloxy ether:mp 115.5-116.5°C; nmr $\tau$ 4.52-4.86 ($H_6$,m), 5.18-5.42 ($H_{2'}$,m), 5.67-6.92 and 6.33 ($H_{3\alpha}$ and $2H_{6'}$, br m, and $CO_2CH_3$ superimposed s), 9.00 ($C_{19}CH_3$,s), 9.08 ($C_{21}CH_3$, d,J $\simeq$ 6Hz), 9.34 ($C_{18}CH_3$,s).

(C) 24-Hydroxy-3$\beta$-(2'-tetrahydropyranyloxy)chol-5-ene (IV)

Lithium aluminum hydride, 0.255 moles (9.7 g), was slurried in 250 ml dry tetrahydrofuran. The methyl ester (III), 100.4 g (0.212 moles), was dissolved in 250 ml tetrahydrofuran and added to the reducing slurry in about 20 minutes. After heating to reflux (0.5 hr.) the lithium aluminum hydride was decomposed by successive additions of 9.7 ml water, 9.7 ml of 10% aqueous sodium hydroxide and 29.5 ml water. The precipitated aluminum salts were filtered and washed with tetrahydrofuran. Concentration of the filtrate to dryness gave a quantitative yield of the desired alcohol (IV):mp 138.5-140.0°C; nmr $\tau$ 4.50-4.90 ($H_6$,br m), 5.14-5.45 ($H_{2'}$,m), 5.73-6.93 ($H_{3\alpha}$ ,$C_{22}CH_2$,$2H_{6'}$,v br m), 9.00 ($C_{19}CH_3$,s), 9.32 ($C_{18}CH_3$,s).

(D) 24-Tosyloxy-3$\beta$-(2'tetrahydropyranyloxy)chol-5-ene (V)

Tetrahydropyranyloxy alcohol (IV), 94 g (0.211 moles), was dissolved in 135 ml of pyridine with gentle heating. Para toluenesulfonyl chloride, 50.4 g (0.264 moles), was added in one batch to the cooled (< 10°C) steroid solution. A heavy precipitate formed over 30 minutes as the refrigerated mixture turned pink. After 4.5 hours, the entire reaction mixture was diluted with tetrahydrofuran and poured into 3.5 liters of cold water with vigorous agitation. At the end of 30 minutes the heavy white precipitate was collected by suction filtration and washed with 3N hydrochloric acid followed by copious amounts of water. A 97.9% yield of the tosylate (V) was obtained as an oil after attempted crystallization:nmr $\tau$ 2.22 and 2.64 (4ArH,ABq,$J_{AB} \simeq$ 8Hz), 4.50-4.79 ($H_6$,m), 5.14-5.33 ($H_{2'}$,m), 5.80-6.31 and 6.28-6.82 ($H_{3\alpha}$,$C_{22}CH_2$, $2H_{6'}$,v br m), 7.55 ($ArCH_3$,s), 9.01 ($C_{19}CH_3$,s), 9.33 ($C_{18}CH_3$,s).

(E) 25a -Homo-3$\beta$-(2'-tetrahydropyranyloxy)cholenonitrile (VI)

Tosylate (V), 10 g (0.0167 moles), was dissolved in 30 ml dimethylformamide/t-butanol (2:1) with heating, under anhydrous conditions. Potassium cyanide, 0.051 moles (3.3 g), was added in one batch to the stirred, light bronze solution. After heating at reflux for 30 minutes, the dark green solution was poured into ammonium chloride (aqueous, saturated) and extracted with methylene chloride. The combined extracts were concentrated to dryness and chromatographed over silica gel (benzene). Recrystallization from ethyl acetate/methanol gave a 67% yield of the nitrile (VI):mp 144/145-6°C; nmr $\tau$ 4.54-4.84 ($H_6$,m), 5.07-5.34 ($H_{2'}$,m), 5.67-6.93 ($H_{3\alpha}$,$C_{22}CH_2$ and $2H_{6'}$, v br m), 8.99 ($C_{19}CH_3$,s), 9.34 ($C_{18}CH_3$,s).

(F) 27-Nor-25-oxo-cholesterol (VII)

The cholenonitrile (VI), 17.5 g (0.0386 moles), was dissolved in 80 ml benzene and added dropwise to methyl magnesium bromide (66 ml, 2.94 M) in 100 ml benzene. A heavy white precipitate separated as spontaneous refluxing occurred. The mixture was heated at reflux overnight. The canary yellow solution was cooled to 40°C and 200 ml of dioxane/6N sulfuric acid (1:1) was added

slowly. The resulting thick emulsion was refluxed 2 hours. After separating and neutralizing the aqueous phase, it was extracted with ethyl acetate. Combining the organic phases, washing with water and concentrating to dryness gave a pale yellow solid that crystallized from methanol to give a 78.5% yield of the hydroxy ketone (VII):mp 127-8°C; nmr $\tau$ 4.55-4.80 ($H_6$,m), 6.20-6.90 ($H_{3\alpha}$,br m), 7.90 ($C_{26}CH_3$,s), 9.00 ($C_{19}CH_3$,s), 9.07 ($C_{21}CH_3$,d, J $\simeq$ 6$H_z$), 9.33 ($C_{18}CH_3$,s).

(G) 25-Hydroxycholesterol (VIII)

A tetrahydrofuran (20 ml) solution of the hydroxy-ketone (VII), 2.11 g (0.0055 moles), was added in 10 minutes to a stirred solution of methyl magnesium bromide (10 ml, 2.94 M) in 10 ml of tetrahydrofuran. After sitting at room temperature overnight, the mixture was carefully made acidic with dilute hydrochloric acid. After refluxing 0.5 hour the product was precipitated by pouring into cold water. 25-Hydroxycholesterol (VIII) was collected by suction filtration (70-80% yield):mp 170.5/173-174.5°C; nmr $\tau$ 4.54-4.79 ($H_6$,m), 6.19-6.89 ($H_{3\alpha}$,br m), 8.79 ($C_{26,27}CH_3$,s), 9.01 ($C_{19}CH_3$,s), 9.34 ($C_{18}CH_3$,s).

While the invention has been described with reference to certain specific embodiments thereof, it is understood that it is not to be so limited since alterations and changes may be made therein which are within the full intended scope of the appended claims.

WHAT IS CLAIMED IS:

1. A process for the preparation of 25-hydroxycholesterol comprising:

(a) reacting 3 β -hydroxy-5-cholenic acid with a lower alkanol in the presence of an inert solvent and acid catalyst thereby forming an ester of cholenic acid,

(b) protecting the 3-hydroxy group of the ester by reacting the ester with a base stable protecting compound in the presence of an inert solvent and acid catalyst,

(c) reacting the ester with a metal hydride in the presence of an aprotic solvent thereby reducing the ester to the corresponding alcohol,

(d) converting the alcohol with a leaving group,

(e) reacting the leaving group with a cyanide in an aprotic solvent thereby forming the corresponding nitrile,

(f) reacting the nitrile with a methyl organometallic compound in an inert solvent and hydrolyzing under acid conditions thereby forming the corresponding methyl ketone and regenerating the 3-hydroxy group, and

(g) reacting the methyl ketone with a methyl organometallic reagent in an inert solvent thereby forming 25-hydroxycholesterol.

2. A process for the preparation of 25-hydroxycholesterol comprising:

(a) reacting 3 β -hydroxy-5-cholenic acid with methanol in the presence of an inert solvent and acid catalyst thereby forming the methyl ester of cholenic acid,

(b) protecting the 3-hydroxy group of the ester by reacting the ester with dihydropyran in the presence of an inert solvent and acid catalyst,

(c) reacting the ester with lithium aluminum hydride in the presence of an aprotic solvent thereby reducing the ester to the corresponding alcohol,

(d) reacting the alcohol with para toluenesulfonyl chloride in the presence of pyridine thereby forming the tosylate,

(e) reacting the tosylate with cyanide in an aprotic solvent thereby forming the corresponding nitrile,

(f) reacting the nitrile with methyl lithium in an inert solvent and hydrolyzing under acid conditions thereby forming the corresponding methyl ketone and regenerating the 3-hydroxyl group, and

(g) reacting the methyl ketone with methyl lithium in an inert solvent thereby forming 25-hydroxycholesterol.

3. In a process of preparing 25-hydroxycholesterol the step of reacting:

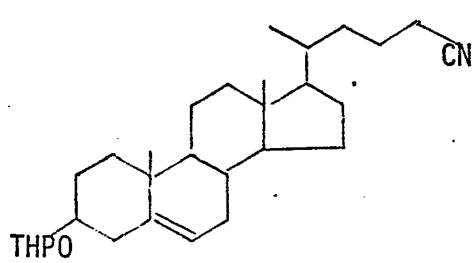

with methyl organometallic reagent in an inert solvent and hydrolyzing under acid conditions thereby forming the corresponding methyl ketone and regenerating the 3-hydroxyl group.

4. The process of claim 3 in which the methyl organometallic reagent is methyl lithium.

European Patent Office

0021235

# EUROPEAN SEARCH REPORT

Application number

EP 80103223.6

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| | DE - A1 - 2 830 019 (KAISER) (25-01-1979)<br>+ Pages 10-17 +<br>-- | 1,2 | C 07 J 9/00 |
| | GB - A - 1 476 992 (HOFFMANN - LA ROCHE)<br>+ Page 2 +<br>-- | 1 | |
| | GB - A - 1 286 761 (UPJOHN)<br>+ Page 4 +<br>---- | 1,2 | |
| | | | TECHNICAL FIELDS SEARCHED (Int.Cl. 3) |
| | | | C 07 J |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 08-09-1980 | PETROUSEK |